(19) Europäisches Patentamt
European Patent Office
Office européen des brevets

(11) **EP 2 796 165 B1**

(12) **FASCICULE DE BREVET EUROPEEN**

(45) Date de publication et mention
de la délivrance du brevet:
**20.04.2016 Bulletin 2016/16**

(51) Int Cl.:
***A61N 1/36*** *(2006.01)*    ***A61N 1/365*** *(2006.01)*

(21) Numéro de dépôt: **13185179.2**

(22) Date de dépôt: **19.09.2013**

(54) **Dispositif médical implantable actif pour le traitement de l'insuffisance cardiaque avec stimulation du nerf vague synchrone à l activité cardiaque**

Aktive implantierbare medizinische Vorrichtung zur Behandlung einer Herzinsuffizienz mit Stimulation des Vagusnervs in Synchronität mit der Herzaktivität

Active implantable medical device for treating heart failure with vagus nerve stimulation synchronised with the heart activity

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **25.04.2013 FR 1353777**

(43) Date de publication de la demande:
**29.10.2014 Bulletin 2014/44**

(73) Titulaire: **Sorin CRM SAS**
**92140 Clamart Cedex (FR)**

(72) Inventeurs:
• **Bonnet, Jean-Luc**
**91300 Massy (FR)**
• **Henry, Christine**
**75014 Paris (FR)**

(74) Mandataire: **Dupuis-Latour, Dominique**
**Bardehle Pagenberg**
**10, boulevard Haussmann**
**75009 Paris (FR)**

(56) Documents cités:
US-A1- 2007 233 194    US-A1- 2010 114 216
US-A1- 2012 209 343    US-A1- 2012 303 080
US-B1- 6 473 644    US-B1- 7 738 956

EP 2 796 165 B1

## Description

L'invention concerne les "dispositifs médicaux implantables actifs" tels que définis par la directive 90/385/CEE du 20 juin 1990 du Conseil des communautés européennes, plus précisément les implants permettant de délivrer des thérapies de stimulation du nerf vague, dites thérapies VNS (*Vagus Nerve Stimulation*).

Elle concerne plus particulièrement l'utilisation de telles thérapies chez des patients en risque d'insuffisance cardiaque.

En effet, la stimulation du nerf vague agit sur les fonctions cardiovasculaires par réduction du rythme cardiaque et de la contractilité du myocarde avec diminution de la durée de la diastole, ce qui peut aider à réduire l'évolution d'un remodelage cardiaque susceptible de mener à une insuffisance cardiaque.

De façon générale, le nerf vague peut être stimulé de façon asynchrone ou bien de façon synchrone avec le rythme cardiaque.

Dans le premier cas, le dispositif est simplement constitué d'une sonde pourvue d'une électrode implantée sur le nerf vague et d'un générateur délivrant des impulsions VNS sur cette électrode, et dans cette configuration il n'y a pas d'interférence possible avec une sonde cardiaque.

En revanche, dans le cas d'une stimulation synchrone, le dispositif comporte en outre une ou plusieurs sondes cardiaques, par exemple une ou plusieurs sondes endocavitaires ou implantées dans le réseau coronarien permettant le recueil des ondes de dépolarisation cardiaque, et éventuellement la délivrance d'impulsions de stimulation du myocarde (stimulation des cavités ventriculaire et/ou auriculaire), en supplément de la stimulation VNS appliquée séparément sur le nerf vague.

Les US 2012/0303080 A1 et US 2007/0233194 A1 divulguent de tels dispositifs de stimulation synchrone du nerf vague.

Dans cette configuration de stimulation VNS synchrone, il convient de délivrer la stimulation dans une période non vulnérable du ventricule.

En effet, si l'on considère un électrocardiogramme de surface ECG ou un électrogramme endocavitaire EGM, parmi les différentes ondes du complexe PQRST représentatif de l'activité cardiaque, on sait que durant le complexe QRS (dépolarisation des ventricules) et l'onde T subséquente (repolarisation des ventricules) le coeur est en période réfractaire. Cette période réfractaire ventriculaire comprend elle-même une période dite "réfractaire absolue", pendant laquelle aucune stimulation électrique n'aura d'action sur les cellules cardiaques, suivie d'une période dite "réfractaire relative", pendant laquelle une stimulation peut entrainer certaines fibres cardiaques et induire une arythmie ventriculaire.

Dans le cas d'une stimulation du nerf vague et dans le cas d'un système avec une sonde ventriculaire, il est communément admis qu'une stimulation VNS délivrée durant cette période réfractaire relative du ventricule est potentiellement délétère, du fait de charges qui pourraient s'accumuler sur l'électrode et déclencher des arythmies ventriculaires. Il convient donc d'éviter de stimuler durant cette période.

Ainsi, les US 2012/0303080 A1 et US 2007/0233194 A1 précités prévoient de synchroniser l'application de la salve d'impulsions VNS sur la détection de l'onde R, de manière à appliquer ces impulsions pendant la période réfractaire ventriculaire absolue qui suit immédiatement cette onde et réduire ainsi les effets délétères potentiels.

Pour ne pas créer de risque pour le patient, une stimulation VNS synchrone doit donc être parfaitement maîtrisée en dépit de la variabilité intrinsèque cycle-à-cycle de l'onde de dépolarisation, en étant impérativement appliquée hors périodes potentiellement délétères.

Le problème de l'invention consiste ainsi à délivrer d'une façon simple une stimulation VNS sans risque d'arythmie cardiaque - et ceci, que le patient fasse ou non l'objet d'une stimulation cardiaque parallèlement à la stimulation VNS.

L'idée de base de l'invention consiste à délivrer la stimulation VNS dans une autre période que la période réfractaire ventriculaire absolue, et sans risque d'arythmie.

Il s'agit en l'espèce de la période correspondant à la fin de l'intervalle d'échappement naturel du ventricule, située après l'onde T (donc bien au-delà des périodes réfractaires ventriculaires), à un moment correspondant à l'apparition de l'onde de dépolarisation auriculaire (onde P) du cycle cardiaque suivant.

Cette proposition tire parti de la double observation que i) l'activité spontanée du nerf vague est principalement concentrée dans l'intervalle cardiaque PQ et ii) la zone précédant la dépolarisation naturelle du ventricule, typiquement dans une fenêtre de quelques dizaines de millisecondes avant l'activité spontanée de celui-ci, peut être considérée comme une période non vulnérable, c'est-à-dire non susceptible de générer des arythmies.

Pour déterminer l'instant d'application de la thérapie VNS dans une telle fenêtre temporelle, la solution la plus simple consisterait à disposer d'une sonde de détection de la dépolarisation auriculaire permettant de détecter la survenue de l'onde P et de caler le début de la délivrance de la salve d'impulsions VNS sur cette détection.

Une telle solution serait toutefois complexe à mettre en oeuvre, nécessitant l'implantation d'une sonde pourvue d'électrodes de détection auriculaire, ainsi qu'une adaptation du générateur, avec un connecteur supplémentaire, des circuits internes dédiés, etc., configuration que l'on ne peut trouver que dans les stimulateurs de type "double chambre".

L'un des buts de l'invention est de proposer une solution à cette difficulté, avec un stimulateur VNS synchronisé sur le signal auriculaire mais qui ne nécessite pas de moyens matériels de détection des dépolarisations auriculaires, notamment pas de sonde additionnelle pour détecter l'onde P sur laquelle la stimulation VNS pourrait être synchronisée.

L'invention pourra ainsi être mise en oeuvre à partir des circuits d'un stimulateur de type "simple chambre", complété par des moyens générateurs de salves d'impulsions VNS déclenchés par des moyens de séquencement appropriés opérant à partir d'une simple sonde de détection ventriculaire.

À cet effet, l'invention propose un dispositif comprenant, de manière en elle-même connue, un générateur produisant des salves d'impulsions VNS, des moyens d'analyse du rythme cardiaque recueillant un signal représentatif de l'activité électrique cardiaque, notamment un signal EGM d'électrogramme endocavitaire et déterminant la durée de cycles cardiaques successifs, et des moyens de séquencement VNS déterminant un instant d'application d'une salve d'impulsions VNS par le générateur.

De façon caractéristique, les moyens de séquencement VNS comprennent des moyens estimateurs calculant lors d'un cycle donné une estimée de la position temporelle de l'onde R du cycle suivant, et des moyens pour définir l'instant d'application de la salve d'impulsions VNS comme étant un instant correspondant à l'estimée calculée de la position temporelle de l'onde R, anticipée d'un délai d'avance estimé.

Dans un mode de réalisation particulier, les moyens d'analyse du rythme cardiaque calculent en outre une durée moyenne, et éventuellement un écart-type, des cycles cardiaques sur une période prédéterminée ou sur un nombre de cycles prédéterminé. Les moyens d'analyse du rythme cardiaque calculent le délai d'avance à partir des durées des cycles cardiaques antérieurs, de manière à refléter la variabilité et l'évolution du rythme cardiaque.

Avantageusement, les moyens d'analyse du rythme cardiaque calculent une durée moyenne des cycles cardiaques sur une période prédéterminée ou sur un nombre de cycles prédéterminé, et les moyens de séquencement VNS calculent l'estimée de la position temporelle de l'onde R en fonction à la fois de ladite durée moyenne et du délai d'avance.

De préférence, le dispositif comprend en outre des moyens de détection d'événements ventriculaires spontanés, et des moyens d'interruption de la délivrance des impulsions produites par le générateur en cas de survenue d'un événement ventriculaire spontané postérieurement à l'instant d'application de la salve d'impulsions VNS. Il est également possible de disposer de moyens de délivrance d'une impulsion de stimulation ventriculaire en cas d'absence de survenue d'un événement ventriculaire spontané à l'expiration d'un intervalle d'échappement prédéterminé.

On va maintenant décrire un exemple de mise en oeuvre de l'invention, en référence aux dessins annexés où les mêmes références numériques désignent d'une figure à l'autre des éléments identiques ou fonctionnellement semblables.

La Figure 1 est une vue générale de présentation du dispositif de l'invention, montrant le générateur, le myocarde et le nerf vague ainsi que les sondes utilisées.

La Figure 2 est une vue schématique par blocs correspondant aux princepales fonctionnalités du générateur du dispositif de l'invention.

La Figure 3 est un chronogramme montrant, sur deux cycles cardiaques successifs, l'onde de dépolarisation cardiaque avec ses différentes périodes caractéristiques et les instants d'application des salves d'impulsions VNS, dans le cas d'une technique connue.

La Figure 4 est homologue de la Figure 3, pour la technique de l'invention.

La Figure 5 est un chronogramme illustrant la variabilité de l'intervalle RR sur une série de cycles cardiaques consécutifs.

Les Figures 6a à 6c sont des chronogrammes illustrant la manière de déterminer l'instant d'application des impulsions de stimulation VNS selon la technique de l'invention dans différentes situations, respectivement : avec détection normale d'une onde R consécutive, avec détection d'une onde R prématurée, et sans détection d'onde R consécutive.

La Figure 7 est un organigramme résumant les différentes étapes de mise en oeuvre de la technique d'application d'une thérapie VNS selon l'invention.

On va maintenant décrire un exemple de réalisation du dispositif de l'invention.

En ce qui concerne ses aspects logiciels, l'invention peut être mise en oeuvre par une programmation appropriée du logiciel de commande d'un stimulateur VNS connu.

Un tel stimulateur comprend un microprocesseur programmable pourvu de circuits pour mettre en forme et délivrer des impulsions de stimulation à des électrodes implantées. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

Le procédé de l'invention est mis en oeuvre par des moyens principalement logiciels, au moyen d'algorithmes appropriés exécutés par un micro-contrôleur ou un processeur numérique de signal. Pour la clarté de l'exposé, les divers traitements appliqués seront décomposés et schématisés par un certain nombre de blocs fonctionnels distincts présentés sous forme de circuits interconnectés, mais cette représentation n'a toutefois qu'un caractère illustratif, ces circuits comprenant des éléments communs et correspondant en pratique à une pluralité de fonctions globalement exécutées par un même logiciel.

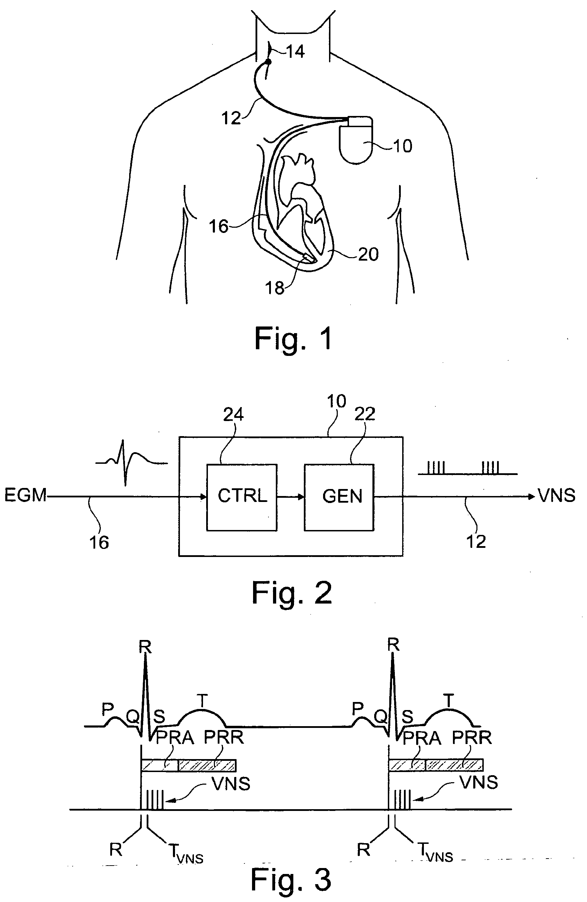

Sur la Figure 1, la référence 10 désigne le boitier d'un générateur implantable de stimulation du nerf vague. Cette stimulation est délivrée par une sonde 12 portant à sa partie distale une électrode implantée sur le nerf vague 14 et susceptible de stimuler celui-ci par application de trains d'impulsions produits par le générateur 10.

Pour permettre la délivrance d'impulsions VNS en synchronisme avec le rythme cardiaque, le générateur 10 dispose également d'une sonde cardiaque 16 pourvue à son extrémité distale d'une électrode 18 de recueil de l'activité électrique du myocarde 20. Cette sonde de recueille des signaux d'électrogramme endocavitaire EGM qui permettront de piloter le générateur 10 afin que celui-ci délivre au nerf vague 14 des impulsions de stimulation VNS au même rythme que les battements cardiaques et au moment le plus approprié de l'onde de dépolarisation cardiaque.

On notera incidemment que l'utilisation d'un EGM endocavitaire n'est pas limitative, et que d'autres techniques peuvent être mises en oeuvre pour obtenir un signal représentatif de l'activité électrique cardiaque.

La Figure 2 illustre de façon schématique les principales fonctionnalités du générateur 10 du dispositif de l'invention.

Celui-ci comprend un circuit générateur 22 apte à produire des salves d'impulsions VNS délivrées au nerf vague via la sonde 12. Le circuit générateur 22 est commandé par un circuit de contrôle 24 lui-même piloté par le signal EGM délivré par la sonde 16.

La Figure 3 illustre un exemple typique, sur deux cycles cardiaques consécutifs, de l'onde de dépolarisation cardiaque recueillie sur un EGM, avec successivement les différentes ondes représentatives de l'activité cardiaque : onde P (dépolarisation des oreillettes), complexe QRS (dépolarisation des ventricules) et onde T (repolarisation des ventricules).

Durant la phase d'activité ventriculaire QRST, le coeur est en période réfractaire, avec une période réfractaire absolue PRA au cours de laquelle aucune excitation, notamment aucune stimulation électrique, n'aura d'action sur les cellules cardiaques, suivie d'une période réfractaire relative PRR pendant laquelle une excitation peut entrainer la dépolarisation de certaines fibres cardiaques. Si l'on doit délivrer une thérapie VNS sous forme d'une salve d'impulsions électriques, l'instant $T_{VNS}$ de délivrance de cette salve, et le nombre et la durée des impulsions de la salve, doivent être tels que les impulsions soient toutes délivrées pendant la période réfractaire absolue PRA. Il s'agit d'éviter tout déclenchement d'arythmie ventriculaire du fait d'une potentielle capture ventriculaire par des champs de courant locaux susceptibles d'entrainer des effets délétères, ce qui pourrait survenir si les impulsions VNS étaient délivrées pendant la période réfractaire relative PRR.

Pour respecter cet impératif, les techniques de stimulation connues opèrent de la manière illustrée Figure 3, en synchronisant l'instant $T_{VNS}$ du début de la salve d'impulsions VNS par rapport à la détection de l'onde R, c'est-à-dire l'instant où la sonde de détection recueille une activité spontanée ayant son origine dans le ventricule. Plus précisément, dans le US 2012/0303080 A1 précité la délivrance de la thérapie est calculée en fonction de l'intervalle PP, tandis que dans le US 2007/0233194 A1 précité, la stimulation VNS est délivrée avec un délai prédéterminé calculé par rapport à l'onde R.

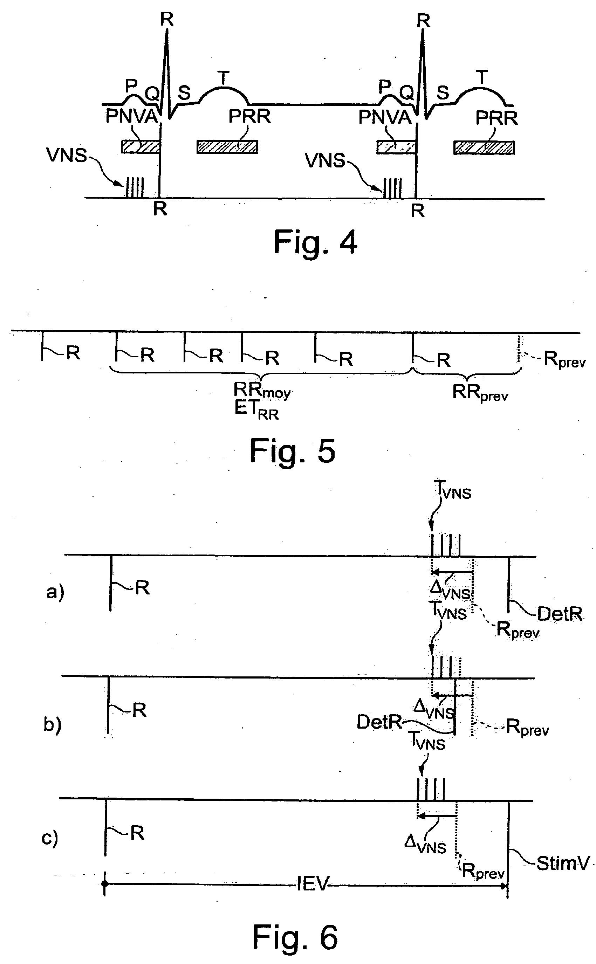

L'invention propose de procéder de manière différente, en délivrant la thérapie VNS au cours d'une autre période du cycle cardiaque, située en dehors des périodes réfractaires ventriculaires naturelles, notamment en dehors de la période réfractaire relative PRR, et sans risque d'arythmie. Pour cela, comme illustré Figure 4 on choisit de délivrer la thérapie VNS à la fin de l'intervalle d'échappement naturel du ventricule, correspondant à une période non vulnérable auriculaire PNVA au cours de laquelle apparait l'onde P de dépolarisation auriculaire, donc bien avant la phase d'activité du ventricule (complexe QRST).

Si l'implant ne dispose pas de moyen de détection de l'activité auriculaire, il convient d'estimer la position temporelle de la prochaine onde ventriculaire et d'anticiper la délivrance de la stimulation VNS par rapport à cette position estimée.

Pour ce faire, comme illustré Figure 5 le dispositif suit l'activité ventriculaire et détermine les instants R de recueil d'une activité ventriculaire spontanée. Sur la base de ces détections, le dispositif calcule la période ventriculaire moyenne $RR_{moy}$ et sa variabilité, correspondant à l'écart-type $ET_{RR}$ du paramètre RR.

En fonction de $RR_{moy}$, et éventuellement de $ET_{RR}$ également, le dispositif calcule un intervalle prévisionnel $RR_{prev}$, par exemple par une fonction du type :

$$RR_{prev} = RR_{moy} - \alpha.ET_{RR}$$

Si $\alpha = 1$, on estime que 85% des cycles RR seront plus longs que $RR_{prev}$, dans le cas d'une distribution gaussienne des intervalles RR.

Ceci permet d'obtenir une estimée $R_{prev}$ de la position temporelle de l'onde R du cycle suivant.

Comme illustré Figure 6a, l'instant $T_{VNS}$ d'application de la salve d'impulsions VNS sera déterminé à partir de cette estimée $R_{prev}$, anticipée d'un délai d'avance $\Delta_{VNS}$, typiquement de l'ordre de $ET_{RR}$ (si $\alpha = 1$).

Dans le cas normal (celui illustré Figure 6a), la délivrance de la thérapie VNS est suivie par la détection d'une activité spontanée DetR, en principe proche de la position estimée $R_{prev}$.

Dans le cas particulier (illustré Figure 6b) d'une activité spontanée DetR prématurée au point de survenir à un instant où la salve d'impulsions VNS n'a pas encore fini d'être délivrée, le dispositif interrompt immédiatement cette délivrance de manière à éviter tout risque de stimulation pendant une période réfractaire ventriculaire.

Dans un autre cas particulier (illustré Figure 6c) où aucun évènement spontané ventriculaire n'a été détecté à l'issue de l'intervalle d'échappement ventriculaire IEV (intervalle compté à partir de la détection R précédente), alors une stimulation ventriculaire StimV est délivrée par le dispositif.

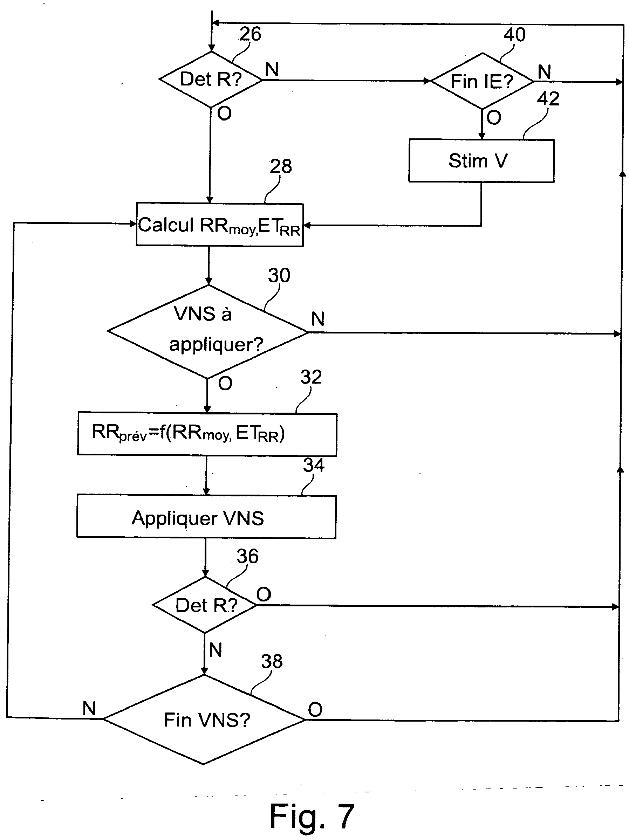

La Figure 7 est un organigramme décrivant l'enchainement des différentes actions que l'on vient de décrire.

Sur détection d'une activité ventriculaire DetR (test 26), le dispositif calcule ou recalcule les valeurs de l'intervalle $RR_{moy}$ et de l'écart-type $ET_{RR}$ (bloc 28).

Si une stimulation VNS doit être appliquée (test 30) alors le dispositif estime la durée prévisible du prochain intervalle RR à partir de la moyenne et de l'écart-type des intervalles RR précédents (bloc 32).

La thérapie VNS est alors appliquée, en déclenchant la délivrance des salves d'impulsions VNS en fonction de l'instant estimé, déterminé à l'étape précédente, de la position temporelle de la prochaine onde R (bloc 34).

Si au cours de la délivrance de la salve d'impulsions une dépolarisation ventriculaire DetR est détectée (test 36), alors cette délivrance est interrompue et le processus est réinitialisé (retour au test 26).

Dans le cas contraire, la délivrance de la thérapie VNS est poursuivie jusqu'à la dernière impulsion de la salve, et le processus est réitéré (retour au bloc 28), à moins que l'on arrive à la fin de la thérapie VNS (test 38), auquel cas le processus est réinitialisé dans sa totalité (retour au test 26). En tout étant de cause, en l'absence de détection DetR (test 26) à la fin de l'intervalle d'échappement ventriculaire (test 40), une stimulation StimV est délivrée au ventricule par le dispositif (bloc 42).

**Revendications**

**1.** Un dispositif médical implantable actif pour le traitement de l'insuffisance cardiaque avec stimulation du nerf vague, VNS, synchrone à l'activité cardiaque, comprenant :

- un générateur (22), apte à produire des salves d'impulsions VNS ;
- des moyens d'analyse du rythme cardiaque, aptes à recueillir un signal représentatif de l'activité électrique cardiaque et à déterminer la durée de cycles cardiaques successifs ; et
- des moyens de séquencement VNS (24), aptes à déterminer un instant d'application d'une salve d'impulsions VNS par le générateur ; dispositif **caractérisé en ce que** les moyens de séquencement VNS comprennent :

- des moyens estimateurs, aptes à calculer lors d'un cycle donné une estimée ($R_{prev}$)

de la position temporelle de l'onde R du cycle suivant ; et
- des moyens pour définir l'instant ($T_{VNS}$) d'application de la salve d'impulsions VNS comme étant un instant correspondant à l'estimée calculée de la position temporelle de l'onde R, anticipée d'un délai d'avance ($\Delta_{VNS}$) estimé.

**2.** Le dispositif de la revendication 1, dans lequel :

- les moyens d'analyse du rythme cardiaque sont en outre aptes à calculer une durée moyenne ($RR_{moy}$) des cycles cardiaques sur une période prédéterminée ou sur un nombre de cycles prédéterminé ; et
- les moyens d'analyse du rythme cardiaque sont en outre aptes à calculer ledit délai d'avance ($\Delta_{VNS}$) à partir des durées des cycles cardiaques antérieurs, de manière à refléter la variabilité et l'évolution du rythme cardiaque.

**3.** Le dispositif de la revendication 2, dans lequel :

- les moyens de séquencement VNS sont aptes à calculer l'estimée ($R_{prev}$) de la position temporelle de l'onde R en fonction à la fois de ladite durée moyenne ($RR_{moy}$) et dudit délai d'avance ($\Delta_{VNS}$).

**4.** Le dispositif de la revendication 1, comprenant en outre :

- des moyens de détection d'événements ventriculaires spontanés (DetR) ; et
- des moyens d'interruption de la délivrance des impulsions produites par le générateur en cas de survenue d'un évènement ventriculaire spontané postérieurement à l'instant d'application de la salve d'impulsions VNS.

**5.** Le dispositif de la revendication 4, comprenant en outre :

- des moyens de délivrance d'une impulsion de stimulation ventriculaire (StimV) en cas d'absence de survenue d'un évènement ventriculaire spontané à l'expiration d'un intervalle d'échappement prédéterminé.

**6.** Le dispositif de la revendication 1, dans lequel le signal représentatif de l'activité électrique cardiaque est un signal EGM d'électrogramme endocavitaire.

**Patentansprüche**

**1.** Aktive implantierbare medizinische Vorrichtung zur Behandlung von Herzinsuffizienz mit Vagusnervstimulation VNS in Synchronität mit der Herzaktivität, umfassend:

- einen Generator (22), der geeignet ist, VNS-Impulssalven zu erzeugen;
- Mittel zur Analyse des Herzrhythmus, die geeignet sind, ein Signal zu erfassen, das für die elektrische Herzaktivität repräsentativ ist, und die Dauer der aufeinander folgenden Herzzyklen zu bestimmen; und
- Mittel zur VNS-Sequenzialisierung (24), die geeignet sind, einen Anwendungszeitpunkt für eine VNS-Impulssalve durch den Generator zu bestimmen;

wobei die Vorrichtung **dadurch gekennzeichnet ist, dass** die Mittel zur VNS-Sequenzialisierung Folgendes umfassen:

- Schätzmittel, die geeignet sind, während eines gegebenen Zyklus einen Schätzwert ($R_{prev}$) der zeitlichen Position der Welle R des folgenden Zyklus zu berechnen; und
- Mittel zum Definieren des Zeitpunkts ($T_{VNS}$) zur Anwendung der VNS-Impulssalve als einen Zeitpunkt, der dem berechneten Schätzwert der zeitlichen Position der Welle R entspricht, dem eine geschätzte vorherige Verzögerung ($\Delta_{VNS}$) vorausgeht.

**2.** Vorrichtung nach Anspruch 1, wobei:

- die Mittel zur Analyse des Herzrhythmus ferner geeignet sind, um eine mittlere Dauer ($RR_{moy}$) der Herzzyklen über einen vorbestimmten Zeitraum oder über eine vorbestimmte Anzahl von Zyklen zu berechnen; und
- die Mittel zur Analyse des Herzrhythmus ferner geeignet sind, die vorherige Verzögerung ($\Delta_{VNS}$) aus Dauern von vorausgehenden Herzzyklen zu berechnen, um die Variabilität und die Entwicklung des Herzrhythmus widerzuspiegeln.

**3.** Vorrichtung nach Anspruch 2, wobei:

- die Mittel zur VNS-Sequenzialisierung geeignet sind, den Schätzwert ($R_{prev}$) der zeitlichen Position der Welle R gleichzeitig sowohl in Abhängigkeit von der mittleren Dauer ($RR_{moy}$) als auch der vorherigen Verzögerung ($\Delta_{VNS}$) zu berechnen.

**4.** Vorrichtung nach Anspruch 1, die ferner Folgendes umfasst:

- Mittel zur Detektion von spontanen ventrikulären Ereignissen (DetR); und
- Mittel zur Unterbrechung der Abgabe der Impulse, die von dem Generator erzeugt werden, beim Eintreten von spontanen ventrikulären Ereignissen nach dem Zeitpunkt der Anwendung der VNS-Impulssalve.

**5.** Vorrichtung nach Anspruch 4, die ferner umfasst:

- Mittel zur Abgabe eines Impulses zur ventrikulären Stimulation (StimV) bei Nicht-Eintreten eines spontanen ventrikulären Ereignisses nach Ablauf eines vorbestimmten Escape-Intervalls.

**6.** Vorrichtung nach Anspruch 1, wobei das Signal, das für die elektrische Herzaktivität repräsentativ ist, ein endokavitäres Elektrokardiogramm-Signal ist.

## Claims

**1.** An active implantable medical device for treatment of heart failure by stimulation of the vagus nerve, VNS, synchronous with the heart activity, comprising:

- a generator (22), adapted to produce bursts of VNS pulses;
- heart rhythm analysis means, adapted to collect a signal representative of the heart electrical activity and to determine the duration of successive heart cycles; and
- VNS sequencing means (24), adapted to determine a time of application of a burst of VNS pulses by the generator;

the device being **characterized in that** the VNS sequencing means comprise:

- estimator means, adapted to calculate during a given cycle an estimate ($R_{prev}$) of the time position of the wave R of the following cycle; and
- means for defining the time ($T_{VNS}$) of application of the burst of VNS pulses as being a time corresponding to the calculated estimate of the time position of the wave R, anticipated by an estimated advance delay ($\Delta_{VNS}$).

**2.** The device of claim 1, wherein:

- the heart rhythm analysis means are further adapted to calculate a mean duration ($RR_{moy}$) of the heart cycles over a predetermined period or over a predetermined number of cycles; and
- the heart rhythm analysis means are further adapted to calculate said advance delay ($\Delta_{VNS}$) based on the durations of the prior heart cycles, so as to reflect the variability and the evolution of the heart rhythm.

3.  The device of claim 2, wherein:

    - the VNS sequencing means are adapted to calculate the estimate ($R_{prev}$) of the time position of the wave R as a function of both said mean duration ($RR_{moy}$) and said advance delay ($\Delta_{VNS}$).

4.  The device of claim 1, further comprising:

    - means for detecting spontaneous ventricular events (DetR); and
    - means for interrupting the delivery of the pulses produced by the generator in case of occurrence of a spontaneous ventricular event posteriorly to the time of application of the burst of VNS pulses.

5.  The device of claim 4, further comprising:

    - means for delivering a ventricular stimulation pulse (StimV) in case of absence of occurrence of a spontaneous ventricular event at the expiry of a predetermined escape interval.

6.  The device of claim 1, wherein the signal representative of the heart electric activity is a signal of endocardial electrogram EGM.

Fig. 1

Fig. 2

Fig. 3

Fig. 4

Fig. 5

Fig. 6

Fig. 7

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20120303080 A1
- US 20070233194 A1